# NEW EUROPEAN PATENT SPECIFICATION

(11) **EP 2 296 728 B2**
(45) Date of publication and mention of the opposition decision: **06.11.2024**
(45) Mention of the grant of the patent: 30.10.2019
(21) Application number: 09737871.5
(22) Date of filing: 29.04.2009
(51) Int. Cl.: A61M 5/142, H03K 17/955, H03K 17/96

(54) **ADMINISTRATION DEVICE HAVING A BUTTON WITH TOUCH SENSOR**
VERABREICHUNGSVORRICHTUNG MIT BERÜHRUNGSSENSOR
DISPOSITIF D'ADMINISTRATION AVEC FONCTIONS DE SÉCURITÉ

(30) Priority: 30.04.2008 EP 08155464
(43) Date of publication of application: 23.03.2011
(73) Proprietor: Roche Diabetes Care GmbH, 68305 Mannheim (DE); F. Hoffmann-La Roche AG, 4070 Basel (CH)
(72) Inventor: FRIEDLI, Kurt, 68259 Mannheim (DE)
(74) Representative: Altmann Stößel Dick Patentanwälte PartG mbB
(86) International application number: PCT/EP2009/003089
(87) International publication number: WO 2009/132827

(56) References cited:
- EP-A1- 1 847 287
- WO-A1-02/064196
- WO-A1-2004/107277
- WO-A1-2006/045523
- WO-A1-2006/045525
- WO-A1-2006/120182
- WO-A1-2007/088444
- WO-A1-2007/088444
- WO-A1-2007/107564
- WO-A1-2007/129254
- WO-A1-2009/070679
- WO-A1-99/07425
- WO-A1-99/07425
- WO-A2-2005/077441
- WO-A2-2005/077441
- WO-A2-2007/126851
- DE-A1- 102004 063 650
- DE-A1- 102004 063 664
- DE-A1- 102006 006 784
- US-A- 4 950 246
- US-A1- 2002 133 113
- US-A1- 2002 133 113
- US-A1- 2006 132 283
- US-A1- 2006 184 123
- US-A1- 2007 021 715
- US-A1- 2007 186 923
- US-A1- 2007 186 923
- US-A1- 2008 033 369
- US-A1- 2008 129 486
- DRUIN ALLISON, HENDLER JAMES: "Robots for Kids: Exploring New Technologies for Learning", MORGAN KAUFMANN PUBLISHERS, 1 January 2000 (2000-01-01), pages 206

## Description

### Technical Field

The present invention relates to an administration device for supplying an injectable or infusible product into an organism, in particular a patient, according to the preamble in claim 1. In particular, the invention relates to an infusion device for therapeutic applications, for example a portable infusion device, which can be adapted for self-administering medicines such as insulin over an extended period of time. The invention is further directed to controlling operation of such an administration device.

### Background Art and Technical Background

Administration devices or self-administration devices comparable to the device in accordance with the present invention are used in particular to serve diabetes patients. An example of a typical device which forms part of the background art is referred to in WO 2006/114288, which is incorporated into the present disclosure by reference for the purpose of disclosing in principle the basic constructive portions which the basic purpose of the device.

Often, an administration device for the self-administration of medicines such as insulin is placed in a patient's pocket and connected to the patient's organism to supply a suitable dose of medicine, in particular insulin, to the patient. The user interface which is provided for the patient usually consists of a display and keys or buttons for entering instructions for a control circuit included in the device. Via the user interface, the administration device may be programmed, information such as the administration history or the remaining medicine amount in a medicine reservoir may be recalled and the administration of medicine boli may be initiated.

Since the administration device usually works automatically through the control of a control device such as a micro controller, it is not necessary for the display to be constantly active. The display requires a lot of electrical energy, hence if the display is in operation for a long time, it is necessary to include large batteries or accumulators in the device. Accordingly, an attempt should be made to activate a display only when it is needed.

In some state of the art devices, the function of activating the display is performed via a dedicated button, wherein providing the additional button is an obstacle to minimize the size of the overall device. Alternatively, the display is activated whenever a button or key is pressed, thus increasing the power consumption.

Administration devices are, for example in diabetes therapy, often carried concealed from view, for example in a trousers pocket, and are often programmed and/or manipulated by pressing keys or buttons through the trousers fabric based on audible and/or tactile feedback signals provided by the administration device. This allows the patient to operate the device and in particular to initiate the administration of medicine boli with the device being concealed from view. If the device is operated in this way, activating the display is unnecessary and undesired due to its energy consumption.

A further problem occurs in case of prior art administration devices which require keys or buttons for programming. Since the administration device is often carried in a clothes pocket, such as a trousers pocket, as described above, it is possible that the keys or buttons are activated arbitrarily and unintended which may result, among others, in an unintended administration of medicine doses. In this context, it has to be taken into account that an administration device in accordance with the prior art, like an administration device in accordance with the invention, has to administer a number of pre-adjustable doses, so called basal doses, which have to be administered in accordance with the therapeutic needs of the patient according to a predefined schedule in a substantially continuous way. In addition, the patient typically needs additional doses, so called bolus doses, depending on particular circumstances and events which will vary from day to day, such that as the correction of high blood glucose values or food intake. It is generally desired to allow administration of such additional doses in an easy and discrete way, using the buttons or keys which are provided in order to operate the device. To prevent the unintended administration of medicine doses which may cause severe medical consequences due to buttons or keys being pressed unintentionally, for example by a key carried in the same pocket as the device, prior-art administration devices are provided with at least two keys or buttons which have to be operated in sequence and within a particular time in order to administer a bolus dose. This means, however, increases the operation complexity and decreases the user comfort in particular if the administration device is operated concealed from view.

A related problem occurs if a button becomes instable due to a defect, e.g. has a loose connection or contact element since this again may cause a large number of unintended operations of a the unstable button, resulting in the administration of a large unintended dose with all its negative consequences. Occurrence of this situation is prevented in prior art devices by providing at least too buttons which have to be operated in sequence as described above.

It is the object of the present invention to improve prior-art administration devices. In particular, the administration device of the present invention should be miniaturised while being safe and convenient to use or should be enabled to consume less energy.

The document US 2002/0133113 A1 discloses a medication delivery device, in particular an injection device, comprising a touch sensitive switch in order to sense if the device is in held in the hand of a user. The document WO 2007/088444 A1 discloses an injection device with a capacitive proximity sensor to detect a skin contact. A similar device is disclosed in WO 2005/077441 A2.

The document WO 99/07425 discloses a delivery device with a proximity sensor between a moveable part and a stationary part of a housing of the device. The switch is mechanically actuated by movement of the moveable part relative to the stationary part, in particular a needle-cover is sliding into the housing. A change of state, e. g. an on/off signal, is provided when the cover is displaced by an object to a rear position in the housing. The switch is located on a side portion of the moveable part and is not getting in touch with the object. The document US 2007/0186923 A1 discloses a drug dispensing device for dispensing a drug dosage to a patient, which may comprise a patient identification reader in form of an electronic fingerprint sensor system on one side of a housing and a dispensing button on an other side of the housing. The document EP 1847287 A1 discloses a liquid ejection device which uses two separate sensors for controlling the inhalation intention of a user. The sensors are represented by a contact detection sensor in form of a mouth switch provided at an inhaling port portion and a hold detection sensor in form of a grip switch provided on an opposite side of the inhaling port portion.

### Disclosure of Invention

In accordance with the present invention, the benefits of the administration device of the present invention are based on a device which is characterised by a touch sensor for generating an activation signal, wherein the touch sensor is incorporated into a button. Four a touch sensor which is arranged in this way, the activation signal is generated if a patient's finger physically touches the button or is close enough to the button such that it can be assumed that the patient intends to operate the button. That is, the same movement of the patient's finger may first cause the touch sensor to generate the activation signal, followed by pressing the button. By discontinuing the motion, the patient may further cause the activation signal to be generated without subsequently pressing the button. The activation signal is not generated if the administration device, in particular a preferably present housing of the administration device, is touched at a position remote from the button.

The term 'key' and 'button' are used in their meaning of an electro-mechanical switching element, wherein a operation element of the key or button must be pressed with a certain fore and/or travel distance for changing the state of the switching element. For this action, the terms 'operating' and 'pressing' are used synonymously. The term 'touch sensor' is used in the meaning of a sensor that may need to be touched to be activated or requires an activation object, such as a finger, in its near proximity, of e.g., about 1mm or below. A touch sensor is referred to as being 'activated' if it generates an activation signal, analogue to a button which is pressed. In contact to a button, operation of a touch-sensor does not involve a mechanical contact and accordingly requires no travel distance to be operated

The present invention is based on the insight that a button of an administration device being pressed directly by an operator or patient, typically by a finger which comes into contact with the button, is representative for a different situation as compared to the button being pressed by a different object, such as a cloths fabric or a key which is stored in the same pocket as the infusion device, and should therefore result in different reactions of the administration device upon the button being pressed.

By means of such a touch sensor which generates an activation signal, it is, in preferred embodiments, possible to activate a display of the device. It is therefore possible to both initiate a device action, such as the administration of a medicine bolus, and to active the display, if the button is operated by a finger, and to initiate the same or a different device action if the button is operated in a different way than by a finger. For this type of preferred embodiment, it is assumed that activating the display is desired if the patient directly accesses the device with a finger while activating the display is not desired if the button is operated by the patient in a different way, for example through clothes. Since most of the energy is not consumed by the display as such in many cases but by a display backlight, the display as such may alternatively be constantly activated, that is, show information, while the display backlight is activated as described above. For this type of embodiment, the term 'activation' in the context of a display refers to the display backlight. This type of embodiment is especially focussed on small energy consumption since the display is only activated when actually needed.

Since there may be situations in which activation of the display is desirable even though the button has been pressed with an object different from the finger, for example with a glove, an additional button for manually activating the display may be present.

In another type of preferred embodiment, the button initiates an action of the device, such as the administration of a medicine bolus, only if the button is pressed by a finger, that is, with the activation signal being generated, and does not initiate this action if the button is operated in a different way, that is, with the activation signal not being generated. This is the case if the button is pressed by further object such as a key carried in the same pocket as the administration device or the button is instable due to a defect. This allows maintaining the safety requirements of prior art devices while allowing actions such as initiating a medicine bolus administration with a single button. In this type of embodiment, the button is enabled by the activation signal. A button is referred to as being 'enabled' if pressing or operating the button results in an action being conducted by the device. In contrast, a button is referred to as being 'disabled' if pressing or operating the button does not result in this action being conducted by the device. This type of embodiment is especially focussed on the device safety with respect to unintended medicine bolus administration in combination with user convenience and discrete oepration.

For clarity reasons, it is assumed here and in the following that the administration of a medicine bolus may be directly initiated by pressing a button of the device. This is the case if medicine boli of fixed amount have to be administered. In some embodiments, however, the button may be pressed repeatedly with each pressing of the button the medicine amount is increased by a given increment. Actual administration of the medicine is only performed if the button has not been pressed for a predefined time. In addition, it may be possible to cancel the administration of a drug bolus before or during administration, e.g., by pressing a further button. Those features are known in the art.

Besides the bolus administration, the button may be used for other instructions, such as general programming or requesting status information of the device, which are also to be conducted only when the button is enabled by the activation signal outputted by the touch sensor.

In accordance with one preferred embodiment of the invention, it is possible to incorporate the touch sensor into the button itself, such that it is not necessary to provide an additional area on the housing for the touch sensor. In addition, the touch sensor is unlikely to be activated for this design mistakenly by the user touching another region of the housing. The housing of the administration device is preferably made of a soft material portion in order on the one hand to enable it to be water-proof, and also for generating an electrical, magnetic or electromagnetic field which can interact with an earthed object, such as an operator's or patient's finger approaching the button. The touch sensor can be a capacitive or inductive sensor arrangement, as available from companies such as ANALOG DEVICE, CYPRESS, MAXIM and others. Furthermore, it is possible to use sensors which work on the basis of changes in resistance or in a conduction value. Another option would be to use light-sensitive sensors for outputting the activation signal.

However, an especially favourable option in accordance with the invention appears to be the capacitive or inductive sensor. This is because it is unlikely in for this design that objects other than the earthed finger of an operator could activate the touch sensor. Further advantages can be achieved by using a control device which enables and/or disables functions of the device within a predetermined time after receiving the activation signal. For instance, if an activation signal is generated by the touch sensor and the button is not operated within a predetermined period of time, the button is disabled because it is very likely that the operator or patient activated the touch sensor unintentionally. On the other hand, if the button has not been operated for a predetermined period of time, it is very likely that the activated display can for example be deactivated, since the patient no longer seems to be currently interested in the information displayed.

According to the invention, a button can be provided with an incorporated touch sensor such that a first action of the device is activated by the non-contact-sensor or touch sensor generating the activation signal while a second action of the device is activated through the button.

For example, the first action may be the activation of a display, screen or backlight of the device and the second action may be administering a medicine dose, such as a medicine bolus. Alternatively, the first action may be the activation of the button and the second action may be the administration of a medicine bolus.

The device according to the invention can also be configured to have a set of multiple buttons and a touch sensor may be incorporated into one or multiple of those buttons. Accordingly, this embodiment of the device may comprise more than one touch sensor.

Furthermore, it may be advantageous if the activation and/or deactivation of the touch sensor and/or button and/or display is communicated to a user or operator of the device, i.e. the patient. For example, it is possible to output a sound signal, for instance when the touch sensor is activated and outputs the activation signal.

It is of course also possible to output signals other than sound signals, for instance visible signals, vibration signals, buzzer signals or the like, which the user or operator will notice even when the device in accordance with the invention is placed in a pocket of the patient's clothing. Furthermore, it might be advantageous if the type of activation is communicated to the operator or patient. For instance, the type of signal outputted in the event of activation may be different for an activation signal which is within a predetermined range of values. If the touch sensor detects a detectable event which is not within a predetermined range, it is possible for a recognisable signal to be outputted in order to inform the operator or patient that something has happened which was close to activating the device to accept instructions inputted using the button. If the event detected by the touch sensor is suitable to allow a signal which is within the predetermined range, the activation signal is outputted and another type of recognisable signal can be brought to the patient's attention. Accordingly, the patient can learn what sort of events can affect the device in an arbitrary manner and should be avoided. Thus, for example, events such as putting keys into the pocket could be identified as actions which should be avoided because the keys could activate the button which is suitable for administering bolus doses.

It is of course also possible to program the controller such that a particular code has to be inputted via the touch sensor first, in order to activate the button or the display or other parts or functions of the device of the invention. For instance, one touch on the touch sensor could activate the display and two touches could activate the button itself, if the touches are applied to the sensor in sequence and within a certain time interval or time period. Any type of "touch codes" could of course be used, providing they are not too complicated for the operator or patient.

It is also possible to use a "touch code" to activate the administration device or a function of the administration device and to then enter data or request data, also by touching or otherwise interacting with the touch sensor.

A method for controlling operation of an administration device comprises the steps of:
a) detecting if a touch sensor is activated, the touch sensor being incorporated into a button, wherein activating the touch sensor generates an activation signal,
b) detecting if the button is being pressed,
c) initiating an action of the device in dependence of the touch sensor being activated or not activated and the button being pressed or not pressed.

In some preferred embodiments of the method, the method comprises the step of administering a medicine bolus if the button is pressed with the activation signal being generated and not administering the medicine bolus if the button is operated in a different way.

In alternatively preferred embodiments of the method, the method comprises the step of activating a display upon the activation signal being generated and of administering a drug bolus upon the button being pressed.

Further aspects as well as preferred and exemplary embodiments of administration devices as well as methods for controlling operation of such devices are given by the dependent claims as well as by the embodiments described below in greater detail.

### Brief Description of Drawings

- Figure 1: shows a schematic representation of a first embodiment of the present invention;
- Figures 2a and 2b: show a second embodiment, which is not part of the invention, in a plan view (Figure 2a) and in a partial sectional view (Figure 2b);
- Figure 3: shows functional parts of a third embodiment;
- Figure 4: shows functional parts of a fourth embodiment;
- Figure 5: shows functional parts of a fifth embodiment; and
- Figure 6: shows functional parts of a sixth embodiment;
- Figure 7a and 7b: show exemplary control flows for embodiments of the invention.

In the drawings, the same reference numbers have been assigned to identical parts or parts which have at least substantially the same function. Accordingly, the respective parts need not be explained anew in connection with each figure, since the explanation of a part in one figure can be applied to other figures.

### Modes for Carrying out the invention

In the following, the administration device in accordance with the different embodiments of the present invention is called an "infusion device" for short.

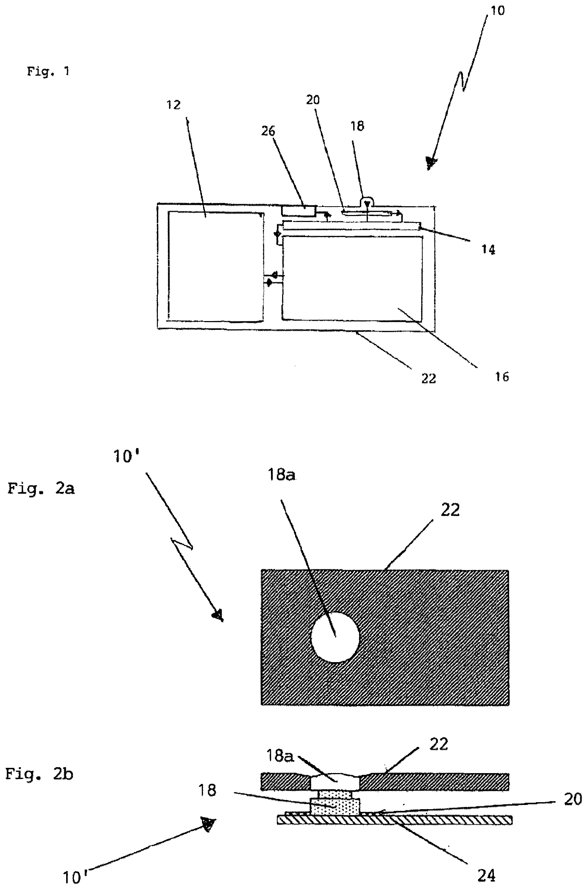

In accordance with Figure 1, an infusion device 10 includes an infusion section 12 which usually consists of a reservoir for a medicine, for example insulin. A supply means, such as a pump, is also provided in order to supply predetermined doses of the medicine through a conduit into the patient's body (not shown). The supply means usually comprises a piston which is accommodated in the reservoir, wherein the reservoir can be replenished. Furthermore, a motor is coupled via a gear to the piston by a piston rod, in order to move the piston in the direction of the centre axis of the reservoir, which usually has a cylindrical shape, to push the medicine out of the reservoir. However, other types of supply means and/or reservoirs may be used as well. For example, the supply means may comprise a micro membrane pump or a peristaltic pump head and/or the reservoir may be formed by a flexible bag, a pouch, or the like.

A power source is usually also included, for supplying power to the motor and other parts of the infusion device 10, such as a controller 14, a display 16, a button 18, a touch sensor 20 incorporated into the button 18 and a printed circuit board 24. The controller 14 also of course includes a processor, a memory portion including a part where non-erasable control data are stored, for instance a ROM memory, and a RAM and/or a reprogrammable memory such as an EEPROM, a flash memory or the like. The controller 14 can be accessed via the button 18, and data or information inputted via the button or requested from the controller can be indicated on the display 16.

A vibrator, a buzzer or loudspeaker or a combination of these can be used to provide a recognisable signal to the operator or patient. Instead of a loudspeaker or buzzer 26, it is of course also possible to use a visual signal which can be emitted by an LED or other device which provides a signal which can be recognised by a person such as a user or patient. The housing 22 of the infusion device 10 accommodates all the different components of the infusion device 10. It can be formed from different materials, for instance plastics or metal, but in the vicinity of the button 18 it is formed from a soft material which does not shield or deflect electrical, magnetic or electromagnetic fields.

In accordance with the invention, the touch sensor 20 is either directly coupled to the button 18 in order to enable or disable the push button 18. In this case, the touch sensor 20 can be considered as being in series with the button 18. Alternatively, the touch sensor 20 is coupled to the controller 14 in order to enable or disable the button 18 via the controller 14. Additionally or alternatively, to enabling or disabling the button 18, the touch sensor 20 can also activate or deactivate the display 16.

When the touch sensor 20 is activated by an object, in particular the earthed finger of an operator or patient, the controller 14 can control the loudspeaker, buzzer, LED 26 or the like to output a recognisable signal. This signal can be used to inform the operator or patient that the button 18 has been enabled in order to input data, request data, or the like, or and/or that the display 16 has been activated to display information, respectively.

Figures 2a and 2b show a second embodiment which is not part of the invention. The top view in Figure 2a shows the housing 22, which is made from a hard plastic material, metal or other suitable material or materials which are resistant to mechanical shocks. A soft material portion 18a is provided, which allows an operator to push the button 18 shown in Figure 2b, and which also enables an electrical field to extend through the soft material portion 18a, such that a person's finger can interact with the electrical field generated by the sensor 20 which, in the present case, can be a capacitive sensor. Accordingly, if an earthed portion of a person's body, for example a finger, approaches the sensor 20 of the infusion device 10', and the interaction is strong enough, the sensor 20 outputs an activation signal which enables the button 18. Both the button 18 and the sensor 20 can be mounted on a printed circuit board which can also support the electrical circuits of the controller 14.

The touch sensor 20 may further be used to input control data, such as an administration schedule, or request the display of data. This could be achieved using a particular code which is inputted by the operator or patient. For instance, the patient approaches the soft material portion two or three times, which is a code for activation, and if the code is accepted, the controller outputs a recognisable signal via the loudspeaker, buzzer or LED 26 shown in Figure 1, in order to inform the patient that the input state has been activated.

It is also for example possible to activate different input states or initiate different actions of the infusion pump using different codes. For example, entering a code may be required for safety-critical operations such as reprogramming or modifying the basal administration schedule.

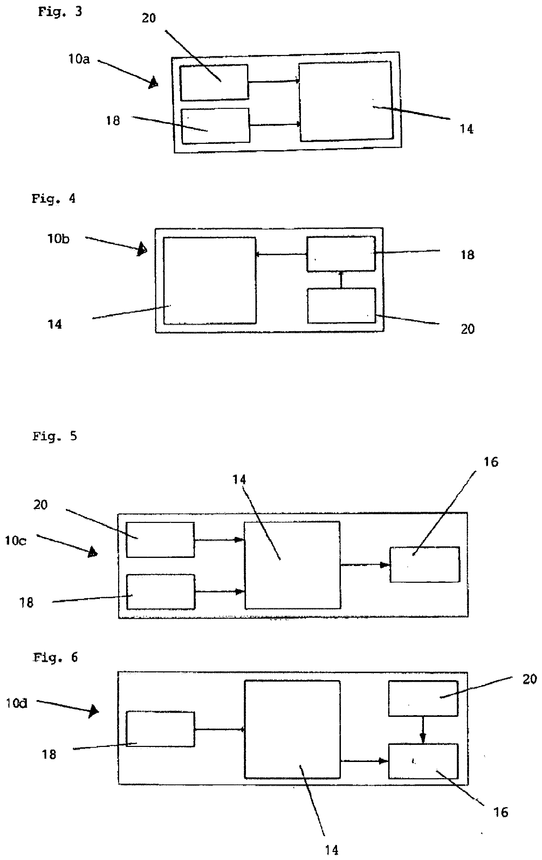

Figure 3 shows only a part of the infusion device 10a, namely the controller 14, the touch sensor 20 and the button or push button 18. In the embodiment of Figure 3, if the sensor 20 outputs an activation signal, the controller 14 enables the button 18 or accepts signals inputted via the button 18 for causing the device to conduct an action, for example a medicine bolus administration. Again, it is possible for the controller 14 to enable the button 18 only for a predetermined but advantageously adjustable period of time of for instance several seconds, in order to prevent an unintended activation of the sensor 20 leading to signals being accepted from the button 18 over a long period of time, which could cause a bolus dose to be unintentionally administered.

In accordance with the embodiment of the device 10b in accordance with Figure 4, an activation signal generated by the sensor 20 directly enables the button or push button 18, thus allowing an operator or patient to input data or requests into the controller 14 via the button 18 and/or to cause the device to conduct an action, for example a medicine bolus administration. Here, the touch sensor 20 and the button 18 can be considered as being electrically arranged in series.

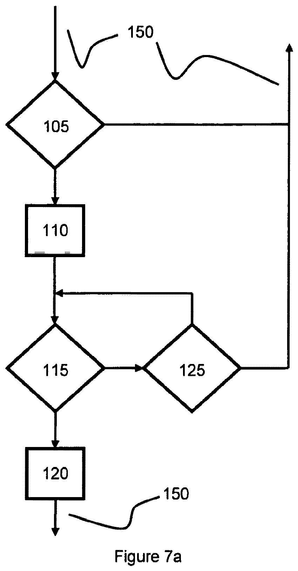

Figure 7a schematically shows a control flow for embodiments according to Figure 3 or Figure 4, respectively. As indicated by the arrows 150 to other parts of the control flow, the steps shown in Figure 7a are carried out repeatedly or substantially continuously in the framework of the overall control flow as controlled by the controller 14. In step 105, it is checked if the touch sensor 20 is activated, that is, if it generates the activation signal. If this is not the case, the general control flow is continued. If the activation signal is generated in step 105, a timer such as a countdown timer is started to enable the button 18 for a predefined period of time, only. In step 110, the display 16 may optionally be activated in addition. In step 115, it is checked if the button 18 is pressed with the activation signal still being generated. If this is not the case, it is checked in step 125 if the timer indicates the predefined period of time for pressing the button 18 has elapsed. If this the case, the general control flow is continued. Otherwise, the step 115 is repeated. If the button 18 is detected to be pressed in step 115, the step of a medicine bolus administration is carried out in step 120 before the general control flow is continued. If the display 16 has been activated in step 110, it preferably switched off or deactivated some time after the medicine bolus administration. In similar embodiments, the steps 110 and 125 may not be present. In this example, the touch sensor 20 is used as a safety frature for enabling the button 18.

In accordance with the embodiment of the device 10c illustrated in Figure 5, the sensor 20 can generate an activation signal which is received by the controller 14. Upon receiving the activation signal from the touch sensor 20, the controller 14 activates a display 16. As also shown in Figure 3, the controller 14 can simultaneously also enable the button 18. In accordance with the embodiment in Figure 5, however, this is merely an option, and it is also possible for the display 16 to be activated only by the activation signal 3 outputted by the touch sensor 20.

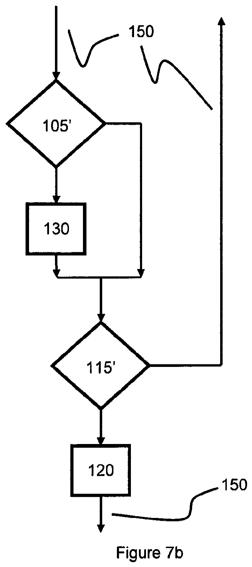

Figure 7b schematically shows a control flow for embodiments according to Figure 5. In step 105', it is checked if the touch sensor 20 is activated, that is, if it generates the activation signal. If this is the case, the display 16 is activated in step 130. Independent of weather the display 16 has been activated or not, it is checked in step 115' if the button 18 is pressed. If this is not the case, the general control flow is continued. If the button 18 is pressed, the step of a medicine bolus administration is carried out in step 120 before the general control flow is continued. In this example, the touch sensor 20 is used to activate the display only if required without having influence on the initiation of a medicine bolus administration via the button 18.

The embodiment in Figure 6 relates to an infusion device 10d in which the touch sensor 20 directly activates the display 16. One or more buttons 18 can be used separately, without influencing the activation signal outputted by the sensor 20. On the other hand, it is of course also possible for the activation signal from the touch sensor 20 to also enable the button or buttons 18 for inputting information or data into the controller 14.

## Claims

1. An administration device (10) for supplying an injectable or infusible product into an organism, in particular a patient,
comprising:
a) a reservoir for storing the product; and
b) a supply device for supplying the product from the reservoir into the organism;
c) a controller (14) configured to control operation of the administration device (10);
d) a button (18) operatively coupled to the controller (14) to initiate at least one action of the administration device (10),
**characterised by**:
e) a touch sensor (20) for generating an activation signal wherein the administration device (10) is configured such that the button (18) is enabled by the activation signal, wherein the touch sensor (20) is incorporated into the button (18) and is operatively coupled to the button (18) and/or the controller (14), wherein the activation signal is generated by the touch sensor (20) if a patient's finger physically touches the button (18) or a finger is in the proximity of the touch sensor (20) of 1mm or below, wherein the controller is configured to enable and/or disable functions of the device within a predetermined period of time after receiving the activation signal.

2. The device according to claims 1, wherein a valid enabling of the button (18) is communicated to a user or operator of the device.

3. The device according to any of the previous claims, wherein the device includes a display (16) which is activated by the activation signal.

4. The device according to any of the previous claims, wherein the touch sensor is a capacitive or inductive sensor.

5. The device according to any of claims 1 to 3, wherein the touch sensor (20) is a sensor which detects a change in resistance, in a conduction value or in light intensity.

6. The device according to any of the previous claims, wherein the device comprises a housing (22) which is formed, at least in the vicinity (18a) of the touch sensor (20), from a material which does not absorb or shield electrical, electromagnetic or magnetic fields.

7. The device according to any of the previous claims, wherein the controller (14) is configured to accept a code inputted via the touch sensor (20) and input data inputted via the sensor (20), once it has received the code.

8. The device according to any of the previous claims, wherein the controller (14) is configured to a first action of the device upon the touch sensor (20) generating the activation signal and to initiate a second action of the device upon the button (18) being pressed.

9. The device according to any of the previous claims, wherein the controller (14) is configured to initiate the administration of a medicine bolus if the button (18) is pressed with the activation signal being generated and not to initiate the administration of a medicine bolus if the button (18) is operated in a different way.

## Patentansprüche

1. Verabreichungsvorrichtung (10) zum Zuführen eines injizierbaren oder infundierbaren Produktes in einen Organismus, insbesondere einen Patienten,
umfassend:
a) einen Behälter zum Lagern des Produktes; und
b) eine Zuführvorrichtung zum Zuführen des Produktes aus dem Behälter in den Organismus,
c) eine Steuerung (14), die konfiguriert ist, um den Betrieb der Verabreichungsvorrichtung (10) zu steuern;
d) eine Taste (18), die funktionsmäßig mit der Steuerung (14) gekoppelt ist, um mindestens eine Aktion der Verabreichungsvorrichtung (10) einzuleiten,
**gekennzeichnet durch**:
e) einen Berührungssensor (20) zum Erzeugen eines Aktivierungssignals, wobei die Verabreichungsvorrichtung (10) so konfiguriert ist, dass die Taste (18) durch das Aktivierungssignal aktiviert wird, wobei der Berührungssensor (20) in die Taste (18) integriert ist und funktionsmäßig mit der Taste (18) und/oder der Steuerung (14) gekoppelt ist, wobei das Aktivierungssignal von dem Berührungssensor (20) erzeugt wird, wenn der Finger eines Patienten die Taste (18) physisch berührt oder sich ein Finger in der Nähe des Berührungssensors (20) von 1 mm oder darunter befindet, wobei die Steuerung konfiguriert ist, um Funktionen der Vorrichtung innerhalb eines vorbestimmten Zeitraums nach dem Empfangen des Aktivierungssignals zu aktivieren und/oder zu deaktivieren.

2. Vorrichtung nach Anspruch 1, wobei eine zulässige Aktivierung der Taste (18) an einen Benutzer oder einen Bediener der Vorrichtung übermittelt wird.

3. Vorrichtung nach einem der vorstehenden Ansprüche, wobei die Vorrichtung eine Anzeige (16) einschließt, die durch das Aktivierungssignal aktiviert wird.

4. Vorrichtung nach einem der vorstehenden Ansprüche, wobei der Berührungssensor ein kapazitiver oder induktiver Sensor ist.

5. Vorrichtung nach einem der Ansprüche 1 bis 3, wobei der Berührungssensor (20) ein Sensor ist, der eine Veränderung des Widerstandes, eines Leitungswertes oder der Lichtintensität erfasst.

6. Vorrichtung nach einem der vorstehenden Ansprüche, wobei die Vorrichtung ein Gehäuse (22) umfasst, das mindestens in der Nähe (18a) des Berührungssensors (20) aus einem Material gebildet ist, das elektrische, elektromagnetische oder magnetische Felder nicht absorbiert oder abschirmt.

7. Vorrichtung nach einem der vorstehenden Ansprüche, wobei die Steuerung (14) konfiguriert ist, um einen Code, der über den Berührungssensor (20) eingegeben wird, und Eingabedaten, die über den Sensor (20) eingegeben werden, zu akzeptieren, sobald sie den Code empfangen hat.

8. Vorrichtung nach einem der vorstehenden Ansprüche, wobei die Steuerung (14) konfiguriert ist, eine erste Aktion der Vorrichtung nach der Erzeugung des Aktivierungssignals durch den Berührungssensor (20) einzuleiten und eine zweite Aktion der Vorrichtung einzuleiten, nachdem die Taste (18) gedrückt wird.

9. Vorrichtung nach einem der vorstehenden Ansprüche, wobei die Steuerung (14) konfiguriert ist, um die Verabreichung eines Medikamentenbolus einzuleiten, wenn die Taste (18) bei erzeugtem Aktivierungssignal gedrückt wird, und die Verabreichung eines Medikamentenbolus nicht einzuleiten, wenn die Taste (18) auf eine andere Weise betätigt wird.

## Revendications

1. Dispositif d'administration (10) pour l'apport d'un produit injectable ou infusible dans un organisme, en particulier un patient,
comprenant :
a) un réservoir pour stocker le produit ; et
b) un dispositif d'alimentation pour apporter le produit depuis le réservoir jusque dans l'organisme ;
c) une commande (14) conçue pour commander le fonctionnement du dispositif d'administration (10) ;
d) un bouton (18) couplé fonctionnellement à la commande (14) pour initier au moins une action du dispositif d'administration (10),
**caractérisé par** :
e) un capteur tactile (20) pour générer un signal d'activation, le dispositif d'administration (10) étant conçu de sorte que le bouton (18) est activé par le signal d'activation, le capteur tactile (20) étant incorporé dans le bouton (18) et étant couplé fonctionnellement au bouton (18) et/ou à la commande (14), le signal d'activation étant généré par le capteur tactile (20) si un doigt du patient touche physiquement le bouton (18) ou si un doigt est à proximité du capteur tactile (20) de 1 mm ou moins, la commande étant conçue pour activer et/ou désactiver des fonctions du dispositif dans une période prédéterminée après la réception du signal d'activation.

2. Dispositif selon la revendication 1, dans lequel une activation valable du bouton (18) est communiquée à un utilisateur ou un opérateur du dispositif.

3. Dispositif selon l'une quelconque des revendications précédentes, dans lequel le dispositif inclut un afficheur (16) qui est activé par le signal d'activation.

4. Dispositif selon l'une quelconque des revendications précédentes, dans lequel le capteur tactile est un capteur capacitif ou inductif.

5. Dispositif selon l'une quelconque des revendications 1 à 3, dans lequel le capteur tactile (20) est un capteur qui détecte un changement de résistance, de valeur de conduction ou d'intensité lumineuse.

6. Dispositif selon l'une quelconque des revendications précédentes, dans lequel le dispositif comprend un boîtier (22) qui est formé, au moins à proximité (18a) du capteur tactile (20), d'un matériau qui n'absorbe pas ou n'abrite pas contre les champs électriques, électromagnétiques ou magnétiques.

7. Dispositif selon l'une quelconque des revendications précédentes, dans lequel la commande (14) est conçue pour accepter un code entré via le capteur tactile (20) et des données d'entrée entrées via le capteur (20) une fois qu'elle a reçu le code.

8. Dispositif selon l'une quelconque des revendications précédentes, dans lequel la commande (14) est conçue pour initier une première action du dispositif une fois que le capteur tactile (20) a généré le signal d'activation et pour initier une seconde action du dispositif une fois qu'on a appuyé sur le bouton (18).

9. Dispositif selon l'une quelconque des revendications précédentes, dans lequel la commande (14) est conçue pour initier l'administration d'un bol de médicament si on a appuyé sur le bouton (18), le signal d'activation étant généré, et pour ne pas initier l'administration d'un bol de médicament si le bouton (18) est actionné de manière différente.
